# EUROPEAN PATENT APPLICATION

(11) **EP 1 016 411 A1**
(43) Date of publication of application: **05.07.2000**
(21) Application number: 99650122.7
(22) Date of filing: 23.12.1999
(51) Int. Cl.: A61K 31/726, A61K 31/727, A61P 43/00

(54) **Sulodexide for restenosis therapy**

(30) Priority: 29.12.1998 IL 12782698
(71) Applicant: Lakaro Biopharmaceutical, Inc., Jerusalem 94383 (IL)
(72) Inventor: Laster, Gail, Jerusalem 97289 (IL)
(74) Representative: McCarthy, Denis Alexis

(57) **Abstract**

A composition for treating restenosis. The composition includes a pharmaceutically effective amount of a composite of heparan or heparin sulfate and dermatan sulfate in particular sulodexide, in combination with a pharmaceutically acceptable carrier.

## Description

### FIELD AND BACKGROUND OF THE INVENTION

The present invention relates to the use of sulodexide in the prevention of restenosis following percutaneous transluminal coronary angioplasty.

Restenosis of blood vessels occurs after narrowed or occluded arteries are forcibly dilated by balloon catheters, drills, lasers and the like, in a procedure known in the art as angioplasty. Restenosis can significantly reduce the long term efficacy of angioplasty and as such is a major barrier to the effective treatment of narrowed arteries.

Smooth muscle and fibroblast cell proliferation and migration is the cardinal event in the pathophysiology of postangioplasty restenosis.

The process of restenosis is thought to be initiated by injury to the arterial wall, followed by a reparative response by the vascular smooth muscle cells. This response consists of migration and proliferation of smooth muscle cells within the arterial intima, leading to the intimal expansion seen in atherosclerotic plaques.

Vascular restenosis or narrowing of the blood vessels, after percutaneous transluminal coronary angioplasty (PTCA) has been shown to be a tissue response characterized by an early and late phase. The early phase, occurring hours to days after PTCA, is due to thrombosis with some vasospasm. The late phase appears to be dominated by excessive proliferation and migration of smooth muscle cells. This along with the formation of connective tissue may be the primary mechanism of the reocclusion of coronary arteries following PTCA, atherectomy, laser angioplasty, arterial bypass graft surgery and heart transplant.

Additional interrelated processes may contribute to restenosis.

These include the injured wall producing new extracellular mass via mechanisms that may be independent of proliferation, retracting of healing tissue in damaged areas and remodeling, a little understood process by which vessels maintain an appropriate lumen size or caliber.

In about 35 % of the patients undergoing PTCA, reocclusion occurs within three to six months after the procedure.

The current strategies for treating vascular restenosis, including mechanical intervention by devices such as stents and pharmacologic therapies, have failed to sufficiently curb the reocclusion rate and thus have been ineffective for the treatment and prevention of vascular restenosis.

Although used extensively, stents alone cannot prevent restenosis caused by neointimal hyperplasia of the tissues of the vessel wall. In fact, the stent material itself may accelerate such hyperplasia, since it is foreign to the body tissues.

More recently, radionuclear irradiation of blood vessels has been proposed as a method of preventing restenosis caused by neointimal hyperplasia. Although it has been shown that procedures relying on irradiating the vessel tissue have been successful at stopping restenosis, methods employing radioactive isotopes have several inherent drawbacks. Currently, radionuclear irradiation of blood vessels is performed by the insertion of temporary or permanent radionuclear sources into the vessels. For example, radioactive yttrium-90 wires were inserted into the central lumen of a balloon catheter in order to irradiate blood vessel walls [Y. Popowski *et al.,* Int. J. Radiation Oncology Biol. Phys., 43:211-215, 1995].

Other radioactive sources have included iridium-192, administered by catheter to arteries which had been treated by angioplasty [P.S. Teirstein *et al.,* Circulation, 94:1-210, 1996].

U.S. Patent No. 5,213,561 discloses a device for inserting a radionuclear source into a blood vessel, in which the source of radioactivity is mounted on a stent, for example.

These devices have a number of disadvantages, as follows.

First, such procedures require a highly restricted clinical setting, which is appropriate both for catheterization procedures and for the handling of radioactivity.

Second, these procedures are highly invasive.

Third, temporary radioactive sources may require repeated invasive treatments.

Thus, current methods for irradiating blood vessels have significant disadvantages. Clearly, methods currently employed in the art, in efforts to combat restenosis, posses serious drawbacks.

There is thus a widely recognized need for, and it would be highly advantageous to have a simple, cost effective method for treatment of restenosis. Such a method can be effected by utilizing a drug capable of specifically arresting or inhibiting the growth of proliferating smooth muscle cells and tissues, the prime contributers to the formation of restenosis. Such a method might also overcome the limitations associated with biomedical device treatments, such as the requirements for specialized clinical setting, etc., and as such, would greatly reduce the cost and hazards typically involved with such treatments.

### SUMMARY OF THE INVENTION

According to one aspect of the present invention there is provided a composition for treating restenosis, the composition comprising a pharmaceutically effective amount of a composite of heparan or heparin sulfate and dermatan sulfate in combination with a pharmaceutically acceptable carrier.

According to another aspect of the present invention there is provided a method of treating restenosis in, for example, a human being, the method comprising the step of administering a composition including a pharmaceutically effective amount of a composite of heparan or heparin sulfate and dermatan sulfate in combination with a pharmaceutically acceptable carrier.

According to yet another aspect of the present invention there is provided a method of manufacturing a medicament for treating restenosis of, for example, a human being, comprising the step of placing a pharmaceutically effective amount of a composite of heparan or heparin sulfate and dermatan sulfate in a pharmaceutically acceptable carrier.

According to further features in preferred embodiments of the invention described below, the composite includes a weight ratio greater than a unit of said heparan or heparin sulfate to dermatan sulfate. Preferably, the composite includes a weight ratio of about 8 to about 2 of the heparan or heparin sulfate and dermatan sulfate.

According to still further features in the described preferred embodiments the composite is sulodexide.

According to still further features in the described preferred embodiments the restenosis is a result of a medical procedure selected from the group consisting of percutaneous transluminal coronary angioplasty, atherectomy, laser angioplasty, arterial bypass graft surgery and heart transplant.

According to still further features in the described preferred embodiments the restenosis is a result of a condition associated with damage or disease inflicted to a vascular epithelial cell layer.

According to still further features in the described preferred embodiments the composition is administered locally, topically, orally, or parenterally.

According to still further features in the described preferred embodiments the pharmaceutically acceptable carrier is adapted for sustained release of the substance.

According to still further features in the described preferred embodiments the pharmaceutically acceptable carrier is a biomedical device adapted for sustained release of the substance.

According to still further features in the described preferred embodiments the biomedical device is selected from the group consisting of a coil, an artificial valve, a vascular graft and a stent.

The present invention successfully addresses the shortcomings of the presently known configurations by providing a new and effective method of treating restenosis.

### DESCRIPTION OF THE PREFERRED EMBODIMENTS

The present invention is of a method of inhibiting restenosis utilizing the anti-fibrinolytic, anti-thrombotic, mild anti-coagulant and anti-proliferative qualities of sulodexide, a glycosaminoglycan composed of heparan or heparin sulfate and dermatan sulfate, wcan be used for treatment of restenosis. Specifically, the present invention can be used to treat restenosis associated with procedures, such as, but not limited to, PTCA, atherectomy, laser angioplasty, arterial bypass graft surgery and heart transplant, or with conditions associated with damage inflicted to the vascular epithelial cell layer, as brought on, by disease etc.

The principles and operation of the present invention may be better understood with reference to the accompanying descriptions and examples.

Before explaining at least one embodiment of the invention in detail, it is to be understood that the invention is not limited in its application to the details of construction and the arrangement of the components set forth in the following description. The invention is capable of other embodiments or of being practiced or carried out in various ways. Also, it is to be understood that the phraseology and terminology employed herein is for the purpose of description and should not be regarded as limiting.

Sulodexide is a glycosaminoglycan composite comprising of approximately 80 % heparan or heparin sulfate and 20 % dermatan sulfate. Native heparan or heparin sulfate serves several functions in endothelial membranes, including anti-proliferative functions, e.g., it suppresses arterial wall smooth muscle cell proliferation, and anti-thrombotic functions, e.g., it activates anti-thrombin III. Dermatan sulfate activates heparan cofactor 2.

Bingley *et al.* (1, 2) showed that heparan sulfate inhibits neointimal proliferation of arterial wall in rabbits *in vitro* as well as *in vivo.*

Bulvas, *et al.* (3) showed that heparan sulfate administration to patients following PTCA due to stenosis of the aorotoiliac or femoropopliteal area improved several angiological parameters.

Indeed, a large, double-blind, multicenter Italian study published in 1990 by Crepaldi, *et al.* (4) revealed a statistically significant improvement in the long term treatment of peripheral vascular disease and a concomitant decrease in plasma triglycerides, fibrinogen and/or viscosity resultant to sulodexide therapy.

Condorelli, *et al.* (5) published a prospective, multicenter, randomized trial assessing the effects of sulodexide in preventing cardiovascular events following myocardial infarction. These researchers found that long-term therapy with sulodexide started early after an episode of acute myocardial infarction is associated with reductions in total mortality, rate of reinfarcation and mural thrombus formation.

Recently, sulodexide was assessed in the treatment of diabetic nephropathy (6-8). Several studies in over 150 patients have shown that sulodexide diminishes urinary albumin excretion which is believed to result from an endothelial defect. The beneficial effect is apparently achieved by supplementing vascular wall heparan sulfate and suppressing mesangial cell proliferation.

Finally, Park, et al. (9) showed that sulodexide was effective in the inhibition of neointimal proliferation after vascular injury in an *in vivo* rat model.

Smooth muscle migration and proliferation are critical processes in the origin and progression of atherosclerotic lesions and restenosis after angioplasty (10). Diseases secondary to atherosclerosis and restenosis are still the most common cause of death in Western industrialized countries. Mounting evidence suggests that a continuous deficiency of glycosaminoglycans on the endothelial surface of blood vessels is one of the underlying pathologic cause.

Sulodexide is well known to have an inhibitory effect on vascular neointimal proliferation. Two decades of clinical usage in the treatment of peripheral vascular disease and diabetic nephotpathy have established a remarkable record of safety for sulodexide in humans.

The connection between an increase in TGFβ and progression of restenosis is well established. Upon an injury to the vascular smooth muscle cells, inactive TGFβ transforms to active TGFβ (11), and, in addition there is a rise in the expression of TGFβ itself and of its receptor (12). Active TGFβ participates in neointimal proliferation and migration of cells (15), and in collagen synthesis and collagen - elastin cross-linking in the extracellular matrix surrounding the cells, thus potentiating the fibrotic process (12). In preliminary experiments it was shown that glycosaminoglycans inhibit TGFβ overexpression under certain conditions (13).

Based on the above, and while conceiving the present invention, it was realized that sulodexide's anti-thrombotic and anti-proliferative actions can find uses in reducing the rate of restenosis after balloon angioplasty as in preventing death and thromboembolic events after acute myocardial infarction.

Thus, according to one aspect of the present invention there is provided a composition for treating restenosis. The composition includes a pharmaceutically effective amount of a composite for treating restenosis. The composite, according to the present invention, includes a composite of heparan or heparin sulfate and dermatan sulfate.

As used herein in the specification and in the claims section below, the terms "heparan or heparin sulfate" and "dermatan sulfate" refer also to derivatives and salts thereof. These sulfates can be of a natural or a synthetic origin. Derivatives of these sulfates, such as, but not limited to, low molecular weight heparan or heparin derivatives obtained by physical, chemical or enzymatic de-polymerization or degradation, chemically modified heparan or heparin derivatives and low molecular weight dermatan sulfates obtained by physical, chemical or enzymatic de-polymerization or degradation, and their preparation, are further described in U.S. Pat. Nos. 5,236,910 and 5,496,807, which are incorporated by reference as if fully set forth herein.

As used herein in the specification and in the claims section below, the terms "treat" and its derivatives includes both pretreatment, before a pathological condition has arisen, and treatment after the condition has arisen. The term "treat" includes both treating the subject after the pathological condition has arisen, and preventing the development of the pathological condition.

As used herein in the specification and in the claims section below, the term "about" refers to ± 20 %.

The composition according to the present invention further includes, in combination with the above described composite, a pharmaceutically acceptable carrier, as further detailed hereinunder.

According to another aspect of the present invention there is provided a method of treating restenosis.

The method according to this aspect of the present invention is effected by administering a composition including a pharmaceutically effective amount of a composite of heparan or heparin sulfate and dermatan sulfate, in combination with a pharmaceutically acceptable carrier. The method can be practiced on a human being suffering from restenosis.

According to yet another aspect of the present invention there is provided a method of manufacturing a medicament for treating restenosis. Manufacturing the medicament according to the present invention is effected by placing a pharmaceutically effective amount of a substance in a pharmaceutically acceptable carrier.

According to a presently preferred embodiment of the present invention the composite includes a weight ratio of greater than a unit, say about 8 parts of heparan or heparin sulfate to about 2 parts of dermatan sulfate, and therefore can be regarded to a sulodexide, which is known to include about these ratios.

As used herein in the specification and in the claims section below, the terms "sulodexide" refers also to derivatives and salts of its components.

Typically, sulodexide is taken to include a mixture of electrophoretically fast migrating heparin sulfate (80 % of the mass) and dermatan sulfate (the balance), with an averaged molecular weight, for most cases, lower 13,000.

Although the following descriptions specifically describe sulodexide as the composite of choice, it will be appreciated by one ordinarily skilled in the art that other closely related preparations, comprising various ratios of heparan or heparin sulfate to dermatan sulfate can as effectively be employed in the treatment of restenosis.

As already mentioned, restenosis is, in many cases the result of a medical procedure, such as, but not limited to, percutaneous transluminal coronary angioplasty, atherectomy, laser angioplasty, arterial bypass graft surgery and/or heart transplant. Alternatively, restenosis is the result of damage or disease inflicted to the vascular epithelial cell layer. It will be appreciated by one ordinarily skilled in the art that the composition, method and medicament according to the present invention are effective in treating restenosis regardless of its cause.

Sulodexide or an equivalent is administered according to an effective dosing methodology, preferably until a predefined endpoint is reached, such as the arrest of restenosis in patients where restenosis is evident or until an experimentally determined preset time, is reached.

Sulodexide or an equivalent can be administered to a subject in a number of ways, which are well known in the art. Hereinafter, the term "subject" refers to the human or lower animal to whom sulodexide was administered.

For example, administration may be done topically (including ophthalmically, vaginally, rectally, intranasally), orally, or parenterally, for example by intravenous drip or intraperitoneal, subcutaneous, or intramuscular injection.

Formulations for topical administration may include but are not limited to lotions, ointments, gels, creams, suppositories, drops, liquids, sprays and powders.

Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets.

Thickeners, diluents, flavorings, dispersing aids, emulsifiers or binders may be desirable.

Formulations for parenteral administration may include but are not limited to sterile aqueous solutions which may also contain buffers, diluents and other suitable additives.

Dosing is dependent on the severity of the symptoms and on the responsiveness of the subject to sulodexide. Persons of ordinary skill in the art can easily determine optimum dosages, dosing methodologies and repetition rates.

According to a preferred embodiment of the present invention the pharmaceutically acceptable carrier is adapted for sustained (also known as slow) release of the composite, so as to affect a prolonged and controlled therapeutic effect thereof.

According to another preferred embodiment of the present invention the pharmaceutically acceptable carrier is a biomedical device, such as, but not limited to, a coil, an artificial valve, a vascular graft or a stent, adapted for sustained release of the composite. The biomedical device can be coated with the composite or alternatively, the composite can be incorporated in a releasable form in the substance from which the device is made.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### Assess the effect of sulodexide on the in vitro proliferation of human arterial smooth muscle cells (SMC)

Human SMC are isolated from the aorta of a second-trimester aborted fetus. These arterial SMC are then cultured and specific criteria for identification by phase contrast and electron microscopy are employed. Well-established measurements of cell growth including ³H-thymidine incorporation into DNA, ³⁵S-methionine incorporation into proteins and separation of the ³⁵S labeled proteins in SDS-Page are utilized to measure SMC proliferation. The extent of inhibition in both exponentially growing and growth arrested cells can be measured. The degree of inhibition at various doses, as well as dose-response curves can be generated.

Based on the above rational, a reversible reduction in the proliferation of SMC in a dose and treatment length dependent manner is expected.

### Assess the ability of sulodexide to inhibit neointimal proliferation after vascular injury

Numerous animal models exist, in which intimal smooth muscle lesions are developed after injury from a balloon catheter. These models show that there is a significant SMC proliferation in the first 72 hours after injury (10). Ten-week-old Sprague Dawley rats subjected to vascular injury by endothelial denudation of the common carotid artery by using a balloon catheter can be used. They are then allocated randomly into (i) a control group, receiving saline, 2 ml, for 3 days, followed by 1 ml for 18 days, intramuscularly; and (ii) an experimental group, given sulodexide, 10 mg/kg/day, for 3 days, and then 4 mg/kg/day for 18 days, intramuscularly. Three weeks after vascular injury, the neointimal proliferation is analyzed using morphometry (9).

Based on the above rational, it is expected that neointimal proliferation will be significantly reduced in the treated group, suggesting that sulodexide is effective in reducing the rate of restenosis after balloon angioplasty.

### In vitro production of TGFβ as a result of treatment with sulodexide

Human SMC are isolated from the aorta of a second-trimester aborted fetus. These arterial SMC are cultured. A mechanical injury is induced thereupon and sulodexide is administrated in vitro. Cell viability is assessed by the tetrazolium salt conversion assay, and active and total TGFβ levels are determined by enzyme linked immunosorbent assay, 24 hours after the treatment. In addition, functional TGFβ activity is assessed by inhibition of endothelial cell mitogenesis (10). A decrease in the TGFβ level and its activity, resulting following sulodexide treatment, is expected.

### Clinical example

Patients undergoing angioplasty receives in loading dose of sulodexide. Following the procedure, patients are administered 100 - 200 mg/day sulodexide for 2 months post - procedure. Follow up is measured by repeat cardiac examinations, such as, but not limited to, angiography, echocardiography and/or ECG. Maintenance of post - angioplasty patency is expected in the treated versus non-treated group after 3 - 6 months.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

### REFERENCES CITED

1. Bingley JA, Campbell JH, Hayward IP et al: Inhibition of neointimal formation by natural heparan sulfate proteoglycans of the arterial wall. Ann. NY Acad Sci 811:238-242, 1997.
2. Bingley JA, Hayward IP, Campbell JH et al: Arterial heparan sulfate proteoglycans inhibit vascular smooth muscle cell proliferation and phenotype change in vitro and neointimal formation in vivo. J Vasc Surg 28(2): 308-318, 1998.
3. Bulvas M, Chochola M, Herdova J et al: The effect of heparin sulfate on percutaneous transluminal angioplasty in the vessels supplying the lower extremities. Cas Kek Cesk 135 (14):445-9, 1996.
4. Crepaldi G, Fellin R, Calabro A, et al: Double-blind multicenter trial on a new medium molecular weight glycosaminoglycan. Atherosclerosis 81:233-43, 1990.
5. Condorelli M, Chiariello M, Digianti A, et al: IPO-V2: A prospective, multicenter, randomized, comparative clinical investigation of the effects of sulodexide in preventing cardiovascular accidents in the first year after acute myocardial infarction. J Am Coll Cardiol 23 (1);27-34, 1994.
6. Poplawska A, Sxelachowska M, Topolska J, et al: Effect of glycosaminoglycans on urinary albumin excretion in insulin-dependent diabetic patients with micro-or macoalbuminuria. Diabetes Res Clin Pract 38(2):109-14, 1997.
7. Solini A, Vergnani L, Ricci F, et al: Glycosaminoglycans delay the progression of nephropathy in NIDDM. Diabetes Care 20: 819-23, 1997.
8. Dedov I, Shestakova, Vorontzov A, et al: A randomized controlled study of sulodexide therapy for the treatment of diabetic nephropathy. Nephrol Dial Transplant 12: 1-6, 1997.
9. Park HY, Kang S, Kim Gy et al: Inhibition of neointimal proliferation of rat carotid artery by sulodexide. J Korean Med Sci 12 (3): 210-14, 1997.
10. Ohno T, Gordon D, San H, et al: Gene therapy for vascular smooth muscle cell proliferation after arterial injury. Science 265:781-84, 1994.
11. Statius van Eps. RG, LaMuraglia GM.: Photodynamic therapy inhibits transforming growth factor beta activity associated with vascular smooth muscle cell injury. J. Vasc. Surg. 25 (6) 1044 - 52, 1997.
12. Shanley CJ, Gharaee - Kermani M, Sarkar R et al.: Transforming growth factor - beta 1 increases lysl oxidase enzyme activity and mRNA in rat aortic smooth muscle cells. J. Vasc. Surg. 25 (3) 446 - 52, 1997.
13. Oturai P, Rolin B, Vissing H: Effects of heparin on kidney TGFβ1 mRNA in experimental diabetic nephropathy. Diabetes 47 (Supl. 1) 120A, 1998.
14. Ward MR, Sasahara T, Agrotis A et al.: Inhibitory effects of tranilast on expression of transforming growth factor - beta isoforms and receptors in injured arteries. Atheroscherosis 137 (2) 267 - 75, 1998.
15. Hornbach V, Waltenberger J, Voisard R et al.: Recurrent stenosis following coronary angioplasty. Cilical, cell biological and molecular aspects. Kardio, 84 (1) 5 - 21, 1995.

## Claims

1. A composition for treating restenosis, the composition comprising a pharmaceutically effective amount of a composite of heparan or heparin sulfate and dermatan sulfate, in combination with a pharmaceutically acceptable carrier.

2. The composition of claim 1, wherein said composite includes a weight ratio greater than a unit of said heparan or heparin sulfate to dermatan sulfate.

3. The composition of claim 1, wherein said composite includes a weight ratio of about 8 to about 2 of said heparan or heparin sulfate and dermatan sulfate.

4. The composition of claim 1, wherein said composite is sulodexide.

5. The composition of claim 1, wherein said restenosis is a result of a medical procedure selected from the group consisting of percutaneous transluminal coronary angioplasty, atherectomy, laser angioplasty, arterial bypass graft surgery and heart transplant.

6. The composition of claim 1, wherein said restenosis is a result of a condition associated with damage or disease inflicted to a vascular epithelial cell layer.

7. The composition of claim 1, wherein the composition is administered locally, topically, orally, or parenterally.

8. The composition of claim 1, wherein said pharmaceutically acceptable carrier is adapted for sustained release of said substance.

9. The composition of claim 1, wherein said pharmaceutically acceptable carrier is a biomedical device adapted for sustained release of said substance.

10. The composition of claim 9, wherein said biomedical device is selected from the group consisting of a coil, an artificial valve, a vascular graft and a stent.

11. A method of treating restenosis, the method comprising the step of administering a composition including a pharmaceutically effective amount of a composite of heparan or heparin sulfate and dermatan sulfate in combination with a pharmaceutically acceptable carrier.

12. The method of claim 11, implemented for treating restenosis of a human being.

13. The method of claim 11, wherein said composite includes a weight ratio greater than a unit of said heparan or heparin sulfate to dermatan sulfate.

14. The method of claim 11, wherein said composite includes a weight ratio of about 8 to about 2 of said heparan or heparin sulfate and dermatan sulfate.

15. The method of claim 11, wherein said composite is sulodexide.

16. The method of claim 11, wherein said restenosis is a result of a medical procedure selected from the group consisting of percutaneous transluminal coronary angioplasty, atherectomy, laser angioplasty, arterial bypass graft surgery and heart transplant.

17. The method of claim 11, wherein said restenosis is a result of a condition associated with damage or disease inflicted to a vascular epithelial cell layer.

18. The method of claim 11, wherein the composition is administered locally, topically, orally, or parenterally.

19. The method of claim 11, wherein said pharmaceutically acceptable carrier is adapted for sustained release of said substance.

20. The method of claim 11, wherein said pharmaceutically acceptable carrier is a biomedical device adapted for sustained release of said substance.

21. The method of claim 20, wherein said biomedical device is selected from the group consisting of a coil, an artificial valve, a vascular graft and a stent.

22. A method of manufacturing a medicament for treating restenosis, comprising the step of placing a pharmaceutically effective amount of a composite of heparan or heparin sulfate and dermatan sulfate in a pharmaceutically acceptable carrier.

23. The method of claim 22, wherein said composite includes a weight ratio greater than a unit of said heparan or heparin sulfate to dermatan sulfate.

24. The method of claim 22, wherein said composite includes a weight ratio of about 8 to about 2 of said heparan or heparin sulfate and dermatan sulfate.

25. The method of claim 22, wherein said composite is sulodexide.

26. The method of claim 22, wherein said restenosis is a result of a medical procedure selected from the group consisting of percutaneous transluminal coronary angioplasty, atherectomy, laser angioplasty, arterial bypass graft surgery and heart transplant.

27. The method of claim 22, wherein said restenosis is of a human being.

28. The method of claim 22, wherein said restenosis is a result of a condition associated with damage or disease inflicted to a vascular epithelial cell layer.

29. The method of claim 22, wherein the composition is administered locally, topically, orally, or parenterally.

30. The method of claim 22, wherein said pharmaceutically acceptable carrier is adapted for sustained release of said substance.

31. The method of claim 22, wherein said pharmaceutically acceptable carrier is a biomedical device adapted for sustained release of said substance.

32. The method of claim 31, wherein said biomedical device is selected from the group consisting of a coil, an artificial valve, a vascular graft and a stent.
